Europäisches Patentamt

European Patent Office

Office européen des brevets

(19)

(11) Publication number: **0 318 286**
**A2**

# EUROPEAN PATENT APPLICATION

(12)

(21) Application number: **88311132.0**

(22) Date of filing: **24.11.88**

(51) Int. Cl.⁴: **C 12 N 5/00**

(30) Priority: **26.11.87 JP 296068/87**

(43) Date of publication of application:
**31.05.89 Bulletin 89/22**

(84) Designated Contracting States:
**BE CH DE ES FR GB IT LI NL SE**

(71) Applicant: **KATAKURA CHIKKARIN CO., LTD.**
**2-3, Otemachi 1-chome Chiyoda-ku**
**Tokyo (JP)**

**KOKEN CO. LTD.**
**5-18 Shimoochiai 3-chome**
**Shinjuku-ku Tokyo (JP)**

(72) Inventor: **Miyata, Teruo**
**2-11-21 Nakane, Meguro-ku**
**Tokyo (JP)**

**Taira, Taira**
**2-11-21 Nakane, Meguro-ku**
**Tokyo (JP)**

**Kimura, Takashi**
**2-3 Otemachi 1-chome**
**Chiyoda-ku, Tokyo (JP)**

**Izume, Masato**
**2-3 Otemachi 1-chome**
**Chiyoda-ku, Tokyo (JP)**

(74) Representative: **Bizley, Richard Edward et al**
**BOULT, WADE & TENNANT 27 Furnival Street**
**London EC4A 1PQ (GB)**

(54) A substratum for cell culture and its production and use.

(57) A novel substratum for cell culture, capable of satisfactorily effecting an initial adhesion and proliferation of cells, is provided which comprises a chitosan-collagen composite material as an effective component.

EP 0 318 286 A2

Description

## A SUBSTRATUM FOR CELL CULTURE AND ITS PRODUCTION AND USE

This invention relates a novel substratum for cell culture and more particularly, it is concerned with a substratum for cell culture of animal cells or plant cells by allowing to adhere thereto and proliferate. The substratum of the present invention can be used for culturing animal or plant cells.

By "substratum for cell culture" described in the present specification is meant a culture medium material such that cells adhere thereto and thus proliferate, namely a cell carrier in the cell culture.

Chitosan is a polysaccharide consisting of N-acetyl-D-glucosamine and D-glucosamine, obtained by deacetylation of chitin contained in shells of crustaceans such as lobsters, crabs, etc. with a concentrated alkali, in which the ratio of N-acetyl-D-glucosamine and D-glucosamine varies with the degree of the deacetylation.

Collagen is a material that is a predominant component of the connective tissue and excellent in biocompatibility and accordingly, it has widely been used as cosmetics or biomaterials. A product has a very low antigenecity and is called atelocollagen, which product is obtained by treating tropocollagen (collagen molecule) with a proteolytic enzyme except collagenase and removing the non-helix part (telopeptide) at the terminal of the molecule.

As the substratum for cell culture, there have hitherto been used various materials or appliances, for example, glass instruments, plastic schales, modified dextran beads, collagen beads, etc. and it has been proposed to use chitosan beads (Japanese Patent Laid-Open Publication No. 51982/1987). Up to the present time, it has also been proposed to use a composite material of an N-acylchitosan and collagen as a biomaterial excellent in biocompatibility, such as wound dressing materials, hemostatic agents, artificial organs, etc. (Japanese Patent Laid-Open Publication No. 253065/1986)

In the culture of cells, plastic schales or petri dishes and chitosan beads provide a relatively fast initial adhesion to the cells, but depending on the variety of the cells, they have poor affinity with the cells such that proliferation of the cells does not proceed well. Collagen beads or collagen films have good affinity with cells, but are inferior in initial adhesion to cells and mechanical strength and more expensive.

The initial adhesion of cells to a material for a substratum for cell culture is important in the case of carrying out cell culture by the microcarrier method and in anchoring-dependent cells, proliferation of the cells cannot be expected without adhering to a material for a substratum for cell culture, while a fast initial adhesion thereto is simul taneously required. In the mass culture of cells, a fast initial adhesion of the cells to a material of a substratum for cell culture is desired. Furthermore, in order to continue a stable culture of cells, it has eagerly been desired to develop a low-priced substratum for cell culture, having good affinity with cells and sufficient mechanical strength in addition to a fast initial adhesion to the cells.

The present invention aims to provide embodiments involving a substratum for cell culture which is capable of satisfactorily effecting an initial adhesion of cells and proliferation of cells. The present invention also aims to provide embodiments in which a substratum for cell culture, which has large mechanical strength, is capable of satisfactorily effecting an initial adhesion of cells and proliferation of cells and can favorably be applied to the culture of animal cells.

Other embodiments aim to provide a process for the production of a substratum for cell culture in an economical manner without superfluous costs, the said substratum having large mechanical strength and being capable of satisfactorily effecting adhesion and proliferation of cells.

These objects can be attained by a substratum for cell culture, comprising a chitosan-collagen composite material as an effective component, and a process for producing the substratum for cell culture which, in one aspect comprises, adding a basic solution to an acidic solution of chitosan in the presence of collagen to precipitate the chitosan and thus forming a chitosan-collagen composite material.

In the accompanying drawings:-

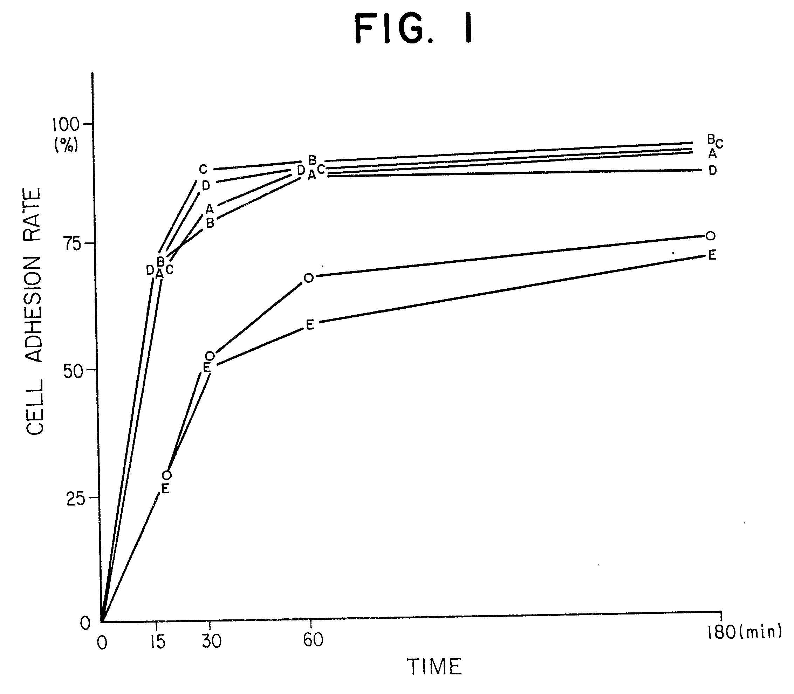

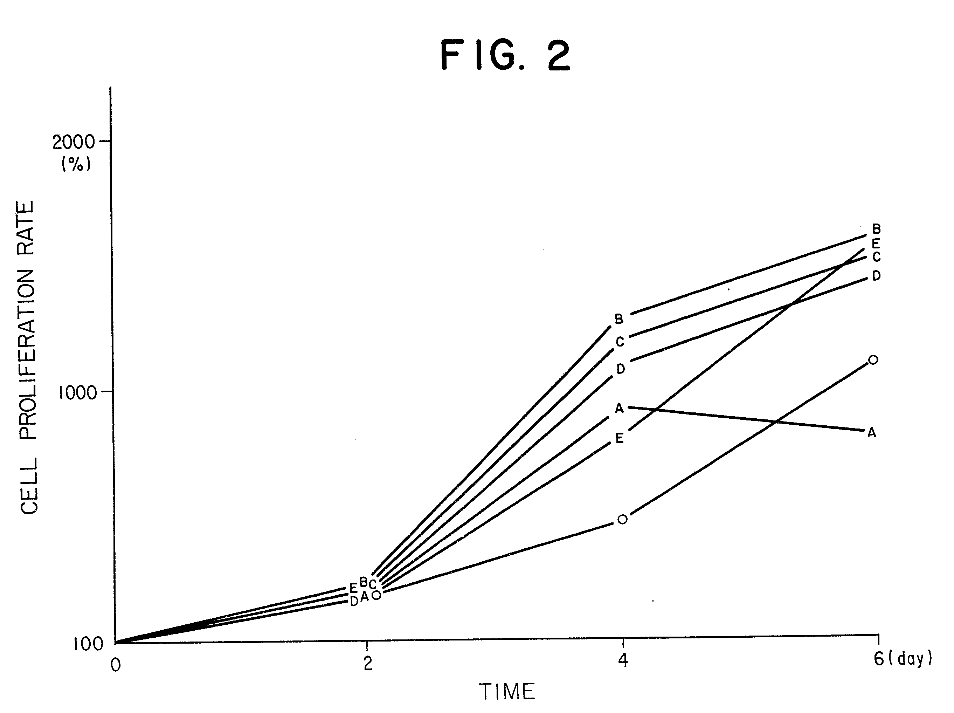

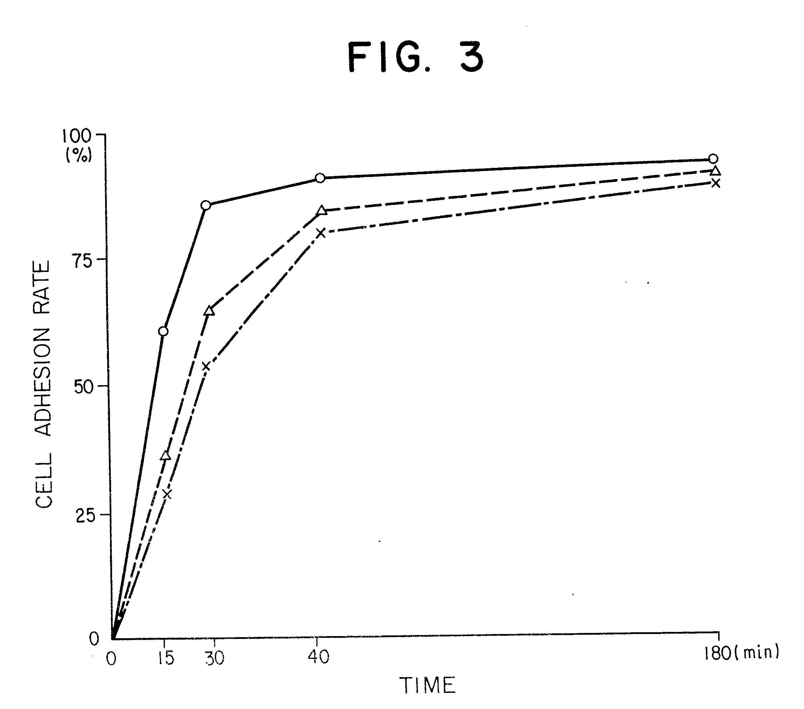

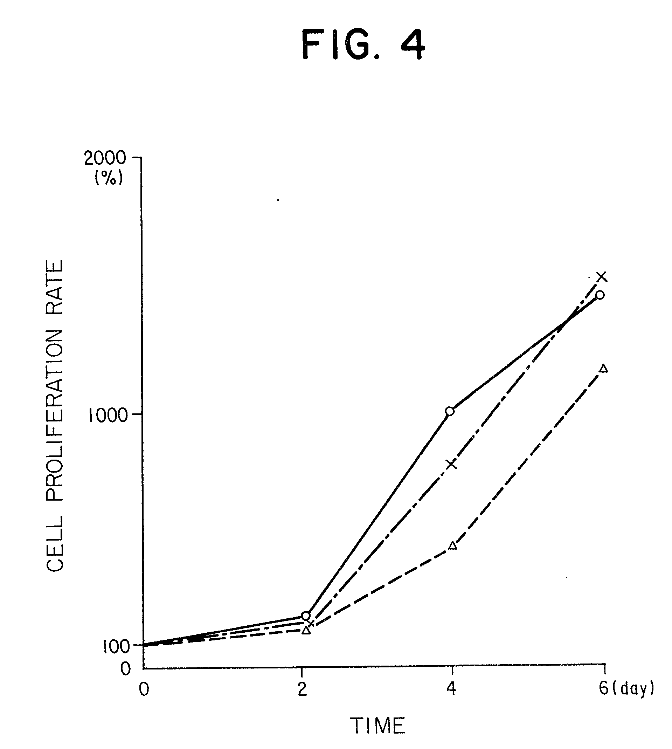

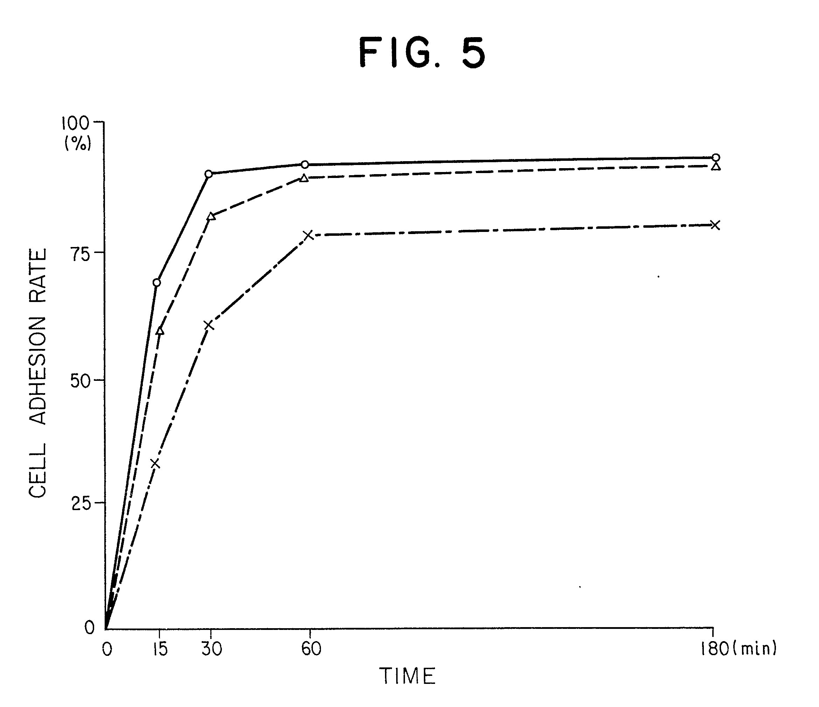

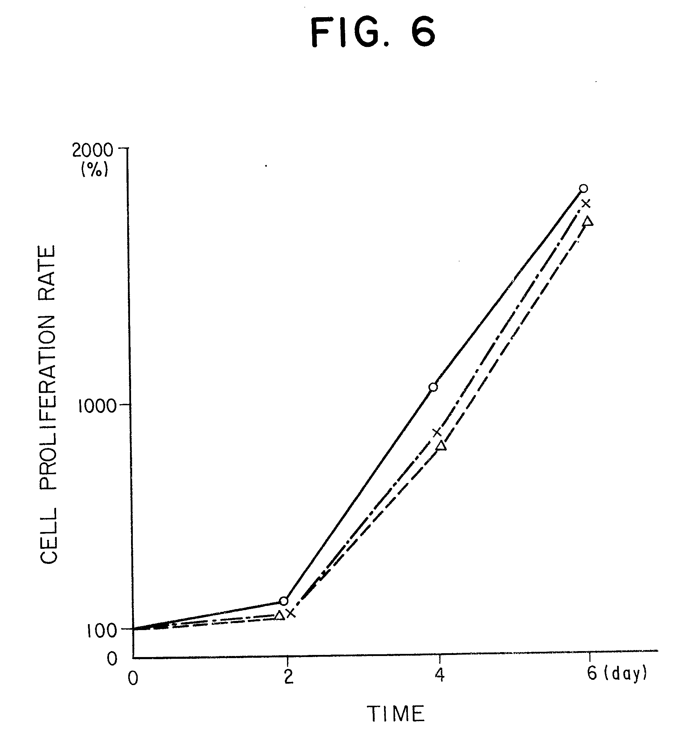

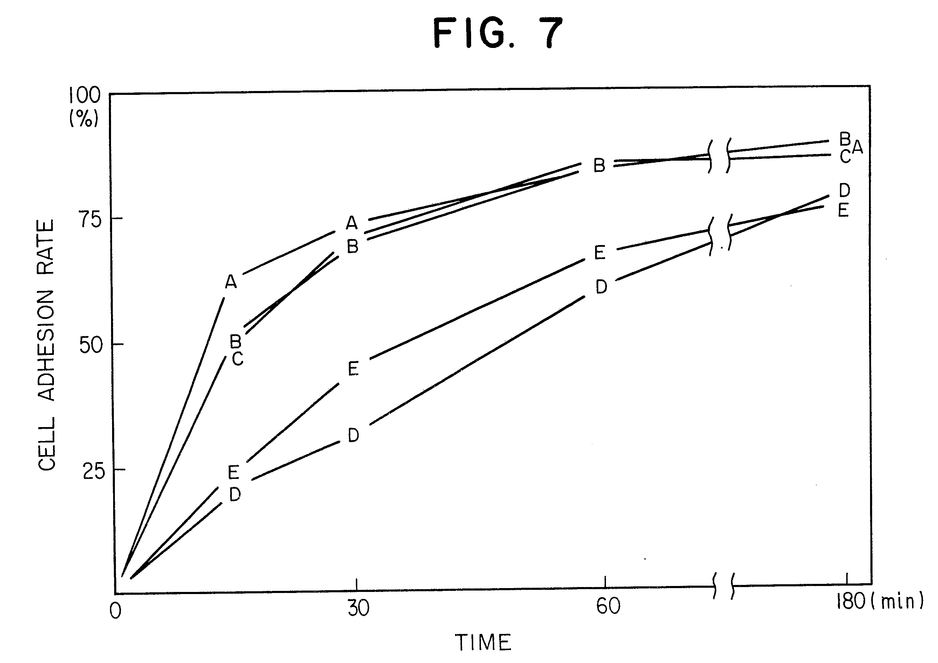

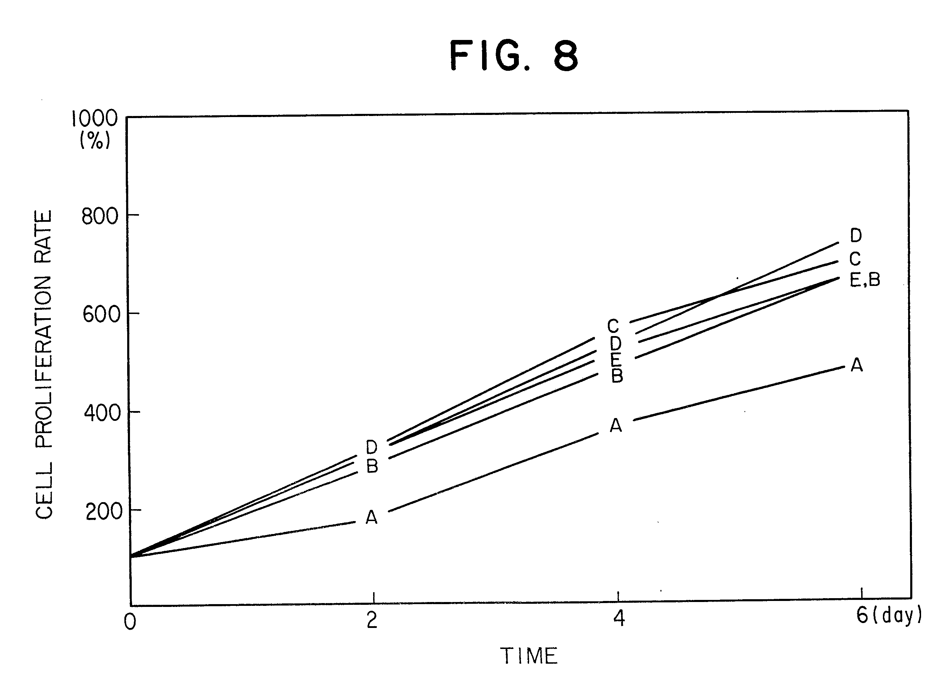

Fig. 1 is a graph showing results of a cell adhesion test of Example 1, Fig. 2 is a graph showing results of a cell proliferation test of Example 1, Fig. 3 is a graph showing results of a cell adhesion test of Example 2, Fig. 4 is a graph showing results of a cell proliferation test of Example 2, Fig. 5 is a graph showing results of a cell adhesion test of Example 3, Fig. 6 is a graph showing results of a cell proliferation test of Example 3, Fig. 7 is a graph showing results of a cell adhesion test and Fig. 8 is a graph showing results of a cell proliferation test.

The inventors have made studies on chitin and chitosan for a long time and consequently, have found in these studies that a chitosan-collagen composite material obtained by combining chitosan and collagen has a sufficient mechanical strength, rapid initial adhesion of cells and good affinity with cells, resulting in a smooth proliferation of the cells. The present invention is based on this finding.

Accordingly, the present invention provides a substratum for cell culture, which has large mechanical strengths and is capable of satisfactorily effecting initial adhesion and proliferation of cells, comprising a chitosan-collagen composite material as an effective component.

The substratum for cell culture of the present invention can be produced by (1) precipitating chitosan and collagen from an acidic solution of chitosan and collagen, (2) adding a basic solution or a solution of a salt capable of precipitating chitosan to an acidic solution of chitosan in the presence of collagen and thus precipitating chitosan, or (3) adding a basic solution or a solution of a salt capable of precipitating collagen to an acidic solution of collagen in the presence of chitosan and thus precipitating collagen.

In the production of the substratum for cell culture comprising a chitosan-collagen composite according to the present invention, the precipitation of chitosan and collagen from an acidic solution of chitosan and collagen can be carried out by removing the solution through evaporation to dryness or by adding a basic solution to the acidic solution to control the pH thereof. As the collagen to be coexistent with the chitosan precipitated from an acidic solution of chitosan, there can be used a shaped product of collagen, and as the chitosan to be coexistent with the collagen precipitated from an acidic solution of collagen, there can be used a shaped product of chitosan. These shaped products of collagen and chitosan may have respectively any suitable form of spherical, rod-shaped, tubular, thin membrane-shaped forms and other forms.

The chitosan-collagen composite material in the substratum for cell culture according to the present invention includes a composite consisting of a uniform mixture of chitosan and collagen, a composite consisting of a chitosan substrate coated with a thin membrane of collagen and a composite consisting of a collagen substrate coated with a thin membrane of chitosan.

Furthermore, the chitosan-collagen composite material in the substratum for cell culture according to the present invention can be subjected to a cross-linking treatment to further increase the mechanical strength and as a crosslinking agent in this treatment, it is preferable to use diisocyanate type compounds.

In the chitosan-collagen composite material of the present invention as a substratum for cell culture, the chitosan includes chitosan, carboxymethylchitosan, glycol chitosan and succinylchitosan. Taking in a narrow sense, this should be said chitosan or its derivatives.

The substratum for cell culture according to the present invention has the advantage that use of collagen in the chitosan-collagen composite material results in good affinity with animal cells and use of chitosan results in increase of the mechanical strength of the substratum for cell culture and the initial adhesion rate of cells.

The chitosan-collagen composite material in the substratum for cell culture according to the present invention consists of chitosan and colalgen in combination and this combination includes homogeneous combinations of chitosan and collagen and heterogeneous combinations of chitosan and collagen.

The chitosan-collagen composite material consisting of a homogeneous combination or mixture can be obtained by mixing an acidic solution of chitosan and an acidic solution of collagen and then evaporating the solvent from the resulting mixed acidic solution or adding to the mixed acidic solution a solution of a basic material, for example, aqueous ammonia to precipitate the chitosan and collagen in the solution phase.

A shaped article or product of the chitosan-collagen composite material can be obtained by adding a mixed acidic solution of chitosan and collagen to a hydrophobic organic solvent such as toluene, followed by stirring to form a system in which the mixed acidic solution phase of chitosan and collagen is dispersed in the form of numerous droplets in the hydrophobic organic solvent phase, then adding to this system a solution of a basic material, for example, aqueous ammonia, followed by stirring to precipitate the chitosan and collagen from the mixed acidic solution phase of chitosan and collagen and taking out of the hydrophobic organic solvent phase, thus obtaining bead-shaped product.

When a mixed acidic solution of chitosan and collagen is extruded in a solution of a basic material through a nozzle with a desired shape to precipitate the chitosan and collagen, a linear or thin membrane-shaped product of the chitosan and collagen having a cross section corresponding to the shape of the nozzle can be obtained. Furthermore, a membrane-shaped article of the chitosan-collagen composite material can also be obtained by charging a mixed acidic solution of chitosan and collagen in a flat bottom vessel, to which a solution of a basic material is then added, and stirring the mixture to precipitate the chitosan and collagen.

In the production of the shaped articles of the chitosan and collagen composite material as illustrated above, on the other hand, an acidic solution of chitosan alone or an acidic solution of collagen alone is used instead of the mixed acidic solution of chitosan and collagen to obtain a shaped article of chitosan alone or collagen alone, each having the same shape as described above. When the thus obtained shaped article of chitosan alone is immersed in an acidic solution of collagen to coat the surface of the shaped article of chitosan alone with the acidic solution of collagen and then immersed in a solution of a basic material, for example, aqueous ammonia, the collagen is precipitated on the shaped article of chitosan alone, thus obtaining a shaped article of the chitosan-collagen composite material consisting of the shaped article of chitosan alone, coated with a membrane of collagen precipitated, which corresponds to an example of the heterogeneous combination of chitosan and collagen. When the shaped article of collagen alone is immersed in an acid solution of chitosan and then immersed in a solution of a basic material, moreover, there can be obtained a shaped article of the chitosan-collagen composite material consisting of the shaped article of collagen alone, coated with a thin membrane of chitosan.

Furthermore, in the production of the substratum for cell culture comprising a chitosan-collagen composite according to the present invention, the chitosan-collagen composite material can be applied or coated in the form of thin membrane onto shaped articles as substrates consisting of porous or non-porous inorganic or organic materials, for example, textile fabrics such as non-woven cloths, membrane, films, beads (100 to 400 mμ in diameter), etc. made of ceramics, glasses, metals, natural and synthetic macromolecular materials such as perlite, vermiculite, cotton, hemp, sugars, polyethylene, polypropylene, silicone rubbers, etc. The coating of these substrates with the chitosan-collagen composite material can be carried out by any conventional methods, for example, a method comprising immersing a substrate in an acidic solution of chitosan and collagen and the adding a basic solution to precipitate a chitosan-collagen composite or a method comprising charging an acidic solution of chitosan and collagen in a container as such a substrate and evaporating the solvent to dryness or adding a basic material to the acidic solution to pre cipitate a chitosan-collagen composite. In these cases, a crosslinking treatment can if necessary be carried out.

When the above described shaped article of the chitosan-collagen composite material is reacted with a crosslinking agent, for example, a diisocyanate compound, the chitosan-collagen composite material is subjected to a crosslinking reaction to form a shaped article of the chitosan-collagen crosslinked composite material having a higher mechanical strength than the former shaped article of the chitosan-collagen composite material.

As the chitosan in the chitosan-collagen composite material of the substratum for cell culture according to the present invention, there can be used chitosan and modified chitosans such as carboxymethylchitosan, glycol chitosan and succinylchitosan. In these chitosan derivatives, glycol chitosan is dissolved in an acidic solution independently of the degree of substitution, but succinylchitosan and carboxymethylchitosan exhibit varied solubility in an acidic solution depending on the degree of substitution, so it is preferable to use those having such a low degree of substitution as to give a higher solubility in an acidic solution.

As the collagen in the chitosan-collagen composite material of the substratum for cell culture according to the present invention, there are generally used neutral salt soluble collagens and acid soluble collagens which can be obtained by extracting a young animal tissue respectively with an aqueous solution of a neutral salt and an aqueous solution of a dilute acid. Furthermore, succinylcollagen and methylated collagen can be used in addition to an enzyme solubilized collagen (atelocollagen) which is made soluble by treating with a proteolytic enzyme such as pepsine an insoluble collagen that is not soluble in the above described extracting procedure.

In the chitosan-collagen composite material of the substratum for cell culture according to the present invention, the proportion of the chitosan and collagen is not particularly limited, but it is preferred to use the composite material containing collagen in a proportion of 5 to 50 % by weight in the form of a solution and 25 to 90 % by weight in the form of a film or bead.

In particular, the adhesion effect is more given in a case where collagen is contained in a proportion of 25 to 90 % by weight and the proliferation effect is more given in a case where collagen is contained in a proportion of 60 to 90 % by weight.

The shaped article of the chitosan-collagen composite material for the substratum for cell culture according to the present invention is preferably in any form of beads, yarns, hollow fibers, non-woven cloths and films. These shaped articles can be made of not only the chitosan-collagen composite material itself but also the other supporting materials as described above.

Examples of the crosslinking agent used in the foregoing crosslinking treatment of the chitosan-collagen composite material for the substratum for cell culture according to the present invention are diisocyanates such as hexamethylene diisocyanate, epihalohydrins such as epichlorohydrin, glutaraldehyde, water-soluble carbodiimides, dicyclohexylcarbodiimide, Woodward reagent and EEDQ (N-ethoxycarbonyl-2-ethoxy-1,2-dihydroquinoline). The crosslinking treatment can generally be carried out by irradiating ultraviolet rays.

The substratum for cell culture according to the present invention is suitable for the culture of animal cells and more specifically is available for the mass culture of cells in the production of interferon, tumor cell antigen, urokinase, insulin, monoclonal antibody and vaccine.

The following examples are given in order to illustrate the present invention in detail without limiting the same. Percents as to the concentration of solutions are generally to be taken as those by weight.

## Example 1

### Preparation of Polymer Coated Schale

A 0.3 % hydrochloric acid solution of atelocollagen (pH: 3.0) and a 0.3 % acetic acid solution of chitosan (pH : 4.0) were mixed in a proportion of the former to the latter as shown in Table 1 to prepare a polymer mixed solution and 1 ml of this solution was poured in a polystyrene schale (Falcon 3001 -commercial name-) having a diameter of 35 mm and a depth of 10 mm. After allowing to stand for 1 hour, the polymer mixed solution was discharged and the polystyrene, schale was rinsed wit sterilized water two times and with a Dulbecco's phosphate buffer solution one time to prepare a polymer coated schale.

### Table 1

Weight Proportion of Chitosan and Atelocollagen in Polymer Mixed Solution

| Sample No. | Chitosan | Atelocollagen |
|---|---|---|
| 1 - A | 100 | 0 |
| 1 - B | 75 | 25 |
| 1 - C | 50 | 50 |
| 1 - D | 25 | 75 |
| 1 - E | 0 | 100 |

### Cell Adhesion Test

Chick embryo fibroblast was suspended in a Dulbecco's Modified Eagle Medium containing 10 % fetal bovine serum in a concentration of $20 \times 10^4$

cells/ml. Then, 1.5 ml of the cell suspension was poured in the polymer coated schale, charged in a $CO_2$ culture apparatus and subjected to incubation at 37 °C for a period of time as shown in Fig. 1. Thereafter, the cell suspension in the polymer coated schale was discharged to count the number of the chick embryo fibroblast (K) in the discharged cell suspension and the difference of the number of the chick embryo fibroblast (U) in the cell suspension first poured in the polymer coated schale from K was defined as the number of the chick embryo fibroblast adhered to the polymer coated schale. Thus, the cell adhesion rates (%) of the chick embryo fibroblast to the above described polymer coated schales are calculated by the following formula:

Adhesion Rate (%) = (U - K)/(U) x 100

The results are shown in Fig. 1, in which Curve (-A-) shows the result of Sample No. 1-A (chotosan = 100), Curve (-B-) shows that of Sample No. 1-B (chotosan/atelocollagen = 75/25), Cure (-C-) shows that of Sample No. 1-C (chotisan/atelocollagen = 50/50), Curve (-D-) shows that of Sample No. 1-D (chitosan/atelocollagen = 25/75), Curve (-E-) shows that of Sample No. 1-E and Curve (-o-) shows that of the non-treated polystyrene schale for control.

Cell Proliferation Test

Chick embryo fibroblast was suspended in a Dulbecco's Modified Eagle Medium containing 10 % fetal bovine serum in a concentration of $4 \times 10^4$ cells/1.5 ml. Then, 1.5 ml of the cell suspension was poured in the polymer coated schale (number of chick embryo fibroblast cells in cell suspension: U), charged in a $CO_2$ culture apparatus and subjected to incubation at 37 °C for a period of time as shown in Fig. 2. After discharging the cell suspension from the polymer coated schale and washing with 1 ml of a Dulbecco's phosphate buffer solution, 1 ml of a 0.1 % trypsin- 10 mM EDTA solution was added thereto and incubated at 37 °C for 10 minutes. Thereafter, the proliferated chick embryo fibroblast cells were detached from the inner wall of the polymer coated schale and rinsed by adding 1 ml of the Dulbecco's phosphate buffer solution. The number of the chick embryo fibroblast cells (M) in the solution was counted to calculate the proliferation rate (%) of the chick embryo fibroblast cells adhered to the polymer coated schale by the following formula:

Proliferation Rate (%) = (M)/(U) x 100

The results are shown in Fig. 2, in which Curves (-A-), (-B-), (-C-), (-D-), (-E-) and (-o-) similarly show results to Fig. 1.

According to the results of Fig. 1, it will clearly be understood that chick embryo fibroblast more adheres to the schales coated with chisosan and the chitosan-collagen composite material than to the non-treated and atelocollagen-coated schales.

According to the results of Fig. 2, it will clearly be understood that chick embryo fibroblast exhibits a larger proliferation rate in the schales coated with the chitosan-atelocollagen composite material and atelocollagen than in the non-treated and chitosan-coated schales.

Example 2

Preparation of Membrane of Glycol Chitosan-Atelo-Collagen Crosslinked Composite Material

100 parts by weight of a 1 % atelocollagen hydrochloric acid solution (pH: 3.0) and 100 parts by weight of a 1 % glycol chitosan acetic acid solution (pH: 4.0) were mixed to prepare a mixed solution of glycol chitosan-atelocollagen and 16 ml of this mixed solution was poured in a polystyrene schale (Falcon 1029) -commercial name-, diameter: 100 mm, depth: 15 mm) and air-dried to form a thin membrane layer of glycol chitosan-atelocollagen composite material on the inner bottom of the polystyrene schale. The resulting thin membrane layer of glycol chitosan-atelocollagen composite material was detached from the polystyrene schale, immersed in a solution of methanol-ammonia (4 : 1) to remove the acids, adequately washed with methanol and then immersed in methanol containing 2 % of hexamethylene diisocyanate to effect a crosslinking reaction for 2 hours. The thin film of the glycol chitosan-atelocollagen crosslinked composite material was adequately washed with methanol and then with water to prepare a finished thin membrane of the glycol chitosan-atelocollagen crosslinked composite material.

Cell Adhesion Test

The above described thin membrane of the glycol chitosan-atelocollagen crosslinked composite material was spread in a polystyrene schale (Falcon 3002 -commercial name-, diameter: 60 mm, depth 15 mm) and a glass ring (outer diameter: 45 mm, inner diameter: 25 mm, height: 5 mm) was placed on the thin membrane, in which 0.75 ml of a cell suspension was poured which had been obtained by suspending chick embryo fibroblasts in a Dulbecco's Modified Eagle Medium containing 10 % fetal bovine serum in a concentration of $20 \times 10^4$ cells/ml. This polystyrene schale was charged in a $CO_2$ culture apparatus and subjected to incubation at 37 °C for a period of time as shown in Fig. 3. The supernatant liquid was taken therefrom to count the number of the chick embryo fibroblast (K) in the supernatant liquid. The difference of the number of the chick embryo fibroblasts (U) in the cell suspension first poured in the polystyrene schale from K, namely (U - K) was defined as the number of the chick embryo fibroblasts adhered to the thin membrane of the glycol chitosan-atelocollagen crosslinked composite material and the adhesion rate (%) was calculated by the following formula:

Adhesion Rate (%) = (U - K)/(U) x 100

On the other hand, a thin membrane of atelocollagen crosslinked material was prepared in the same manner as described above but using only the 1 % atelocollagen hydrochloric acid solution (pH: 3) without using the 1 % glycol chitosan acetic acid solution (pH: 4.0), and using this thin membrane of atelocollagen crosslinked material, the adhesion rate (%) of the chick embryo fibroblasts was obtained in the same manner as described above.

Furthermore, a glass ring (outer diameter: 45 mm, inner diameter: 25 mm, height: 5 mm) was directly placed on a polystyrene schale (Falcon 3002 -commercial name-) without using the thin membrane of the glycol chitosan-atelocollagen crosslinked composite material and other procedures were carried out in the same manner as described above, thus obtaining the adhesion rate (%) of the chick embryo fibroblasts.

The results are shown in Fig. 3, in which Curve (-o-) shows the adhesion rate of the chick embryo fibroblast to the thin membrane of the glycol chitosan-atelocollagen crosslinked composite material, Curve (-x-) shows that in the case of the thin membrane of atelocollagen crosslinked material and Curve (-Δ-) shows that in the case of the polystyrene schale (Falcon 3002).

Cell Proliferation Test

The above described thin membrane of the glycol chitosan-atelocollagen crosslinked composite material was spread in a polystyrene schale (Falcon 3002, diameter: 60 mm, depth: 15 mm) and a glass ring (outer diameter: 45 mm, inner diameter: 25 mm, height: 5 mm) was placed on the thin membrane, in which 0.75 ml of a cell suspension was poured which had been obtained by suspending chick embryo fibroblast cells in a Dulbecco's Modified Eagle Medium containing 10 % fetal bovine serum in a concentration of $2 \times 10^4$ cells/0.75 ml (the number of chick embryo fibroblast cells in the cell suspension poured in the polystyrene schale: U). This polystyrene schale was charged in a $CO_2$ culture apparatus and subjected to incubation at 37 °C for a period of time as shown in Fig. 4.

After taking out the supernatant liquid, the thin membrane of the glycol chitosan-atelocollagen crosslinked composite material in the polystyrene schale was washed with 1 ml of a Dulbecco's phosphate buffer solution, to which 1 ml of a 0.1 % trypsin - 10 mM EDTA solution was added, followed by incubation at 37 °C for 10 minutes. Thereafter, the proliferated chick embryo fibroblast cells were detached from the thin membrane of the glycol chitosan-atelocollagen crosslinked composite material and washed with Dulbecco's phosphate buffer solution. The number of the chick embryo fibroblast cells (M) in the washing solution was counted to calculate the proliferation rate (%) of the chick embryo fibroblasts adhered to the thin membrane of the glycol chitosan-atelocollagen crosslinked composite material by the following formula:

Proliferation Rate (%) = (M)/(U) x 100

On the other hand, a thin membrane of atelocollagen crosslinked material was prepared in the same manner as described above except using the 1 % atelocollagen hydrochloric acid solution (pH : 3.0) without using the 1 % glycol chitosan acetic acid solution (pH : 4.0), and using this thin membrane of atelocollagen crosslinked material, the proliferation rate (%) of the chick embryo fibroblasts was obtained in the same manner as described above.

Furthermore, a glass ring (outer diameter: 45 mm, inner diameter: 25 mm, height: 5 mm) was directly placed on a polystyrene schale (Falcon 3002) without using the thin membrane of the glycol chitosan-atelocollagen crosslinked composite material and other procedures were carried out in the same manner as described above, thus obtaining the proliferation rate (%) of the chick embryo fibroblast.

The results are shown in Fig. 4, in which Curve (-o-) shows the proliferation rate of the chick embryo fibroblast in the thin membrane of the glycol chitosan-atelocollagen crosslinked composite material, Curve (-x-) shows that in the thin membrane of the atelocollagen crosslinked material and Curve (-Δ-) shows that in the polystyrene schale (Falcon 3002).

According to the results of Fig. 3 and Fig. 4, it will clearly be understood that the use of the glycol chitosan-atelocollagen crosslinked composite material results in a better cell adhesion rate and cell proliferation rate than in the case of not using them.

Example 3

Preparation of Thin Membrane of
Carboxymethylchitosan-Atelocollagen Crosslinked
Composite Material

100 parts by weight of a 1 % atelocollagen hydrochloric acid solution (pH: 3.0) was mixed with 200 parts by weight of a 1 % carboxymethylchitosan (substitution degree of carboxymethyl groups: 0.15) acetic acid solution (pH: 4.5). 16 ml of the resulting mixed solution was poured in a polystyrene schale (Falcon 1029, diameter: 100 mm, depth: 15 mm), allowed to stand and air-dried to form a thin membrane layer of a carboxymethylchitosan-atelocollagen composite material in the polystyrene schale. The thus resulting thin membrane of the carboxymethylchitosan-atelocollagen composite material was detached from the polystyrene schale, allowed to stand in an atmosphere of ammonia gas for 2 hours, adequately washed with water, immersed in reacted in a 1 % aqueous carbodiimide solution at 30 °C for one night and then adequately washed with water to prepare a thin membrane of a carboxymethylchitosan-atelocollagen crosslinked composite material.

Cell Adhesion Test

A cell adhesion test was carried out in an analogous manner to Example 2 except using the thin membrane of the carboxymethylchitosan-atelocollagen crosslinked composite material as described above instead of the thin membrane of the glycol chitosan-atelocollagen crosslinked composite material in the cell adhesion test of Example 2.

The results are shown in Fig. 5, in which Curve (-o-) shows that cell adhesion rate of the fowl fibroblasts to the thin membrane of the carboxymethylchitosan-atelocollagen crosslinked composite material, Curve (-x-) shows that to the thin membrane of the atelocollagen crosslinked material and Curve (-Δ-) shows that to the polystyrene schale (Falcon 3002).

Cell Proliferation Test

A cell proliferation test was carried out in an analogous manner to Example 2 except using the above described thin membrane of the carboxymethylchitosan-atelocollagen crosslinked composite material instead of the thin membrane of the glycol chitosan-atelocollagen crosslinked composite material in the cell proliferation test of Example 2.

The results are shown in Fig. 6, in which Curve (-o-) shows the cell proliferation rate of the chick embryo fibroblast in the thin film of the carboxymethylchitosan-atelocollagen crosslinked composite material, Curve (-x-) shows that in the thin membrane of the atelocollagen crosslinked material and Curve (-Δ-) shows that in the polystyrene schale (Falcon 3002).

According to the results of Fig. 5 and Fig. 6, it will clearly be understood that the carboxymethylchitosan-atelocollagen crosslinked composite material exhibits an excellent cell adhesion rate as well as cell proliferation rate similarly to the atelocollagen crosslinked material and accordingly, is an excellent substratum for cell culture.

Example 4

Preparation of Succinylchitosan-Atelocollagen Crosslinked Composite Beads

50 ml of a 3 % atelocollagen hydrochloric acid solution was mixed with 150 ml of a 3 % succinylchitosan (succinylation degree: 0.2) acetic acid solution to prepare a succinylchitosan-atelocollagen mixed solution.

400 ml of toluene and 110 ml of chloroform were charged in a beaker with a volume of 1000 ml, to which Span 20 (-commercial name-, polyoxethylene sorbitan monooleate) was then added to prepare a mixed solvent containing 0.1 % by weight of Span 20. To this mixed solvent was added 150 ml of the above described succinylchitosan-atelocollagen mixed solution and vigorously stirred to form a succinylchitosan-atelocollagen mixed solution phase dispersed in the form of numerous droplets in a mixed solvent phase. While stirring the thus formed dispersion phase, aqueous ammonia was added thereto to precipitate a succinylchitosan-atelocollagen composite material, thus forming succinylchitosan-atelocollagen composite beads. The mixed solvent was then separated by decantation from the beaker and the succinylchitosan-atelocollagen composite beads were adequately washed with methanol.

The thus resulting succinylchitosan-atelocollagen composite beads were then added to 500 ml of a 1 % aqueous carbodiimide solution, stirred to disperse the succinylchitosan-atelocollagen composite beads in the solution and further stirred at 30 °C overnight to form succinylchitosan-atelocollagen crosslinked composite beads, which were taken out of the solution, adequately washed with water and classified in the range of 48 to 100 mesh, thus preparing finished succinylchitosan-atelocollagen crosslinked composite beads.

Cell Adhesion and Cell Proliferation Tests

Mouse skin fibroblasts (L-cells) were suspended in a Dulbecco's Modified Eagle Medium containing 10 % fetal bovine serum to prepare a cell suspension, to which the above described succinylchitosan-atelocollagen crosslinked composite beads were added, charged in a $CO_2$ culture apparatus and then subjected to incubation at 37 °C for 5 days. The surfaces of the succinylchitosan-atelocollagen bridged composite beads were substantially covered with the proliferated cells.

Example 5

Preparation of Chitosan-Atelocollagen Composite Hollow Fibers

50 g of a 2 % atelocollagen hydrochloric acid solution (pH: 3.0) and 50 g of a 2 % chitosan acetic acid solution (pH: 4.0) were mixed to prepare a chitosan-atelocollagen mixed solution, which was then extruded from an annular nozzle having a discharge port with an outer cylinder inner diameter of 1.5 mm and inner cylinder outer diameter of 0.7 mm into a 35 % aqueous sodium chloride solution bath at a rate of 4 m/min to form a hollow fiber. In this extrusion, a 35 % aqueous sodium chloride solution was discharged from the inner cylinder of the annular nozzle and used as an inner solution. The solidified hollow fiber (hollow yarn) was irradiated by a 30 W ultraviolet lamp at an interval of 20 cm, immersed in a 0.1 % aqueous sodium carbonate solution containing 5 % of sodium chloride for 5 minutes, then washed with flowing water for 10 minutes in a water washing tank and dried at 40 °C while blowing air into one end of the capillary tube to prepare a chitosan-atelocollagen composite hollow fiber.

Cell Adhesion and Cell Proliferation Tests

Using the above described chitosan-atelocollagen composite hollow fiber, cell adhesion and cell proliferation tests were carried out in an analogous manner to Example 4. In these tests, the chitosan-atelocollagen composite hollow fiber was substantially covered with mice skins fibroblasts similarly proliferated.

Example 6

Preparation of Chitosan-Coated Collagen Beads

30 ml of commercially available collagen beads (Koken Cellgen Microsphere -commercial name-) were added to 100 ml of a 0.3 % chitosan solution and after slowly stirring and dispersing the mixture, the collagen beads were separated by filtration and added to 5 % aqueous ammonia to precipitate chitosan on the surfaces of the collagen beads. The chitosan-adhered collagen beads were washed with water, further washed with methanol, immersed in a 2 % hexamethylene diisocyanate solution, thus reacted at 20 °C for 2 hours, subjected to filtration and then washed with methanol and water in order, thus preparing chitosan-coated collagen beads.

Cell Adhesion and Cell Proliferation Tests

Using the above described chitosan-coated collagen beads, cell adhesion and cell proliferation tests were carried out as to mouse skin fibroblasts in an analogous manner to Example 4.

In these tests, the chitosan coated collagen beads were substantially covered with the proliferated mouse skin fibroblasts.

Example 7

Preparation of Atelocollagen-Coated Chitosan Beads

400 ml of toluene and 110 ml of chloroform were charged in a 1000 ml beaker and to the mixed solvents was added Span 20 to prepare the mixed solvents containing 0.1 % (by weight) of Span 20. 150 ml of a 3 % chitosan acetic acid solution was then added to the mixed solvents and vigorously stirred to form a chitosan solution phase in the form of numerous droplets in the mixed solvent phase. Aqueous ammonia was added thereto with agitation to precipitate chitosan and to form chitosan beads and after separating the mixed solvents by decantation from the beaker, the chitosan beads were adequately washed with methanol.

100 ml of a 0.3 % atelocollagen hydrochloric acid solution (pH: 3.0) was charged in a 300 ml beaker, to which 30 ml of the above described chitosan beads were added, followed by slowly stirring and dispersing the mixture. Then, the chitosan beads were separated by filtration and added to 5 % aqueous ammonia to precipitate atelocollagen on the surfaces of the chitosan beads. The thus atelocollagen-deposited chitosan beads were washed with water, further adequately washed with methanol, then immersed in a 2 % hexamethylene diisocyanate methanol solution to effect a crosslinking reaction for 2 hours and then adequately washed with methanol, thus obtaining atelocollagen-coated chitosan beads.

Cell Adhesion and Cell Proliferation Tests

Using the above described atelocollagen-coated chitosan beads, cell adhesion and cell proliferation tests were carried out as to mouse skin fibroblasts (L-cells) in an analogous manner to Example 6.

In these tests, the atelocollagen-coated chitosan beads were substantially covered with the proliferated mouse skin fibroblasts.

Example 8

Preparation of Chitosan-coated Collagen Beads

An acid-soluble collagen was extracted by immersing a fetal bovine corium in a 0.5 M acetic acid solution for 3 days, filtered by a membrane filter and then precipitated and purified by a salting out method. The thus resulting acid-soluble collagen was dissolved in a physiological buffer solution so as to give a concentration of 1 %, dispersed in toluene to prepare beads with a diameter of 100 to 300 μm, subjected to crosslinking with hexamethylene diisocyanate and then coated with chitosan in an analogous manner to Example 6, thus obtaining chitosan-coated collagen beads.

Example 9

Preparation of Thin Membrane of Chitosan-Atelocollagen Crosslinked Composite Material

A 1 % hydrochloric acid solution of atelocollagen (pH: 3.0) and a 1 % acetic acid solution of chitosan (acetic acid concentration: 1 % by volume) were mixed in a proportion as shown in Table 2 to prepare a polymer mixed solution and 2 ml of this mixed solution was poured in a polystyrene schale (Falcon 3001) having a diameter of 35 mm and a depth of 10 mm. After air-drying, the thin membrane adhered schale was subjected to crosslinking by irradiating by a UV lamp at an interval of 10 cm and further allowed to stand in an atmosphere of ammonia for 20 minutes. The resulting schale was washed with distilled water two times and further washed with a Dulbecco's phosphate buffer solution one time, thus obtaining a schale with a thin membrane of a chitosan-atelocollagen crosslinked composite material formed on the bottom.

Table 2

Weight Proportion of Chitosan and Atelocollagen in Polymer Mixed Solution

| Sample No. | Chitosan | Atelocollagen |
|------------|----------|---------------|
| 9 - A | 30 | 70 |
| 9 - B | 20 | 80 |
| 9 - C | 10 | 90 |
| 9 - D | 0 | 100 |
| 9 - E | 0 | 0 |

Cell Adhesion Test

A cell adhesion test was carried out in an analogous manner to Example 1 except using the above described schale with the chitosan-atelocollagen crosslinked composite membrane on the bottom instead of the polymer coated schale used in Example 1, thus obtaining results as shown in Fig. 7 in which Curve (-A-) shows the result of Sample No. 9-A (chitosan:atelocollagen = 30 : 70), Curve (-B-) shows that of Sample No. 9-B (chitosan:atelocollagen = 20 : 80), Curve (-C-) shows that of Sample No. 9-C (chitosan:atelocollagen = 10 : 90), Curve (-D-) shows that of Sample No. 9-D (chitosan:atelocollagen = 0 : 100) and Curve (-E-) shows that of the non-treated polystyrene schale (Falcon 3001) for comparison.

Cell Proliferation Test

A cell proliferation test was carried out in an analogous manner to Example 1 except using the above described schale with the chitosan-atelocollagen crosslinked composite membrane on the bottom instead of the polymer coated schale used in Example 1, thus obtaining results as shown in Fig. 8 in which Curve (-A-) shows the result of Sample No. 9-A (chitosan: atelocollagen = 30 : 70), Curve (-B-) shows that of Sample No. 9-B (chitosan:atelocollagen = 20 : 80), Curve (-C-) shows that of Sample No. 9-C (chitosan:atelocollagen = 10 : 90), Curve (-D-) shows that of Sample No. 9-D (chitosan: atelocollagen = 0 : 100) and Curve (-E-) shows that of Sample No. 9-E (non-treated polystyrene schale, Falcon 3001).

According to the results of Fig. 7, it will clearly be understood that chick embryo fibroblasts more adhere to the chitosan-atelocollagen crosslinked composite membrane than to the polystyrene schale and atelocollagen thin membrane.

According to the results of Fig. 8, it wil clearly be understood that chick embryo fibroblasts exhibit substantially similar proliferation rates on the chitosan-atelocollagen composite membranes (10 : 90, 20 : 80) to the polystyrene schale and atelocollagen thin membrane, and a larger proliferation rate on the chitosan-atelocollagen composite membranes (10 : 90, 20 : 80) than on the chitosan-atelocollagen composite membrane (30 : 70).

Since the substratum for cell culture according to the present invention has an excellent initial adhesion and proliferation of cells as well as a sufficient mechanical strength, it can favorably be applied to the mass culture of animal cells. Furthermore, production of the substratum for cell culture according to the present invention can readily be carried out by adding a solution of a basic material and thus precipitating.

## Claims

1. A substratum for cell culture, comprising a chitosan-collagen composite material as an effective component.

2. A substratum for cell culture, as claimed in Claim 1, wherein the chitosan-collagen composite material is either a uniform mixture of chitosan and collagen, or comprises a chitosan substrate coated with a thin film layer of collagen, or comprises a collagen substrate coated with a thin film layer of chitosan.

3. A substratum for cell culture, as claimed in Claim 1 or Claim 2, wherein the chitosan-collagen composite material is crosslinked with a crosslinking agent to increase the mechanical strength, the crosslinking agent optionally being selected from diisocyanates, epihalohy-drins, glutaraldehyde, water-soluble carbo-diimide, dicyclohexylcarbodiimide, Woodward reagent and DEDQ.

4. A substratum for cell culture, as claimed in any one of Claims 1 to 3, wherein the chitosan in the chitosan-collagen composite material is selected from chitosan, carboxymethylchitosan, glycol chitosan and succinylchitosan, and/ or the collagen in the chitosan-collagen composite material is selected from succinylcollagen, methylated collagens and enzyme-solubilized collagens.

5. A substratum for cell culture, as claimed in any one of Claims 1 to 4, wherein the ratio of the chitosan and collagen in the chitosan-collagen composite material is 5 to 90% by weight of collagen and 95 to 10% by weight of chitosan.

6. A substratum for cell structure, as claimed in any one of Claims 1 to 5, wherein the chitosan-collagen composite material is shaped in the form of beads, yarns, hollow fibers, nonwoven cloths or films.

7. A process for producing a substratum for cell culture, comprising either: (a) adding a base or salt to an acidic solution of chitosan in the presence of collagen to precipitate the chitosan and thus form a chitosan-collagen composite material; or (b) adding a base or salt to an acidic solution of collagen in the presence of chitosan to precipitate the collagen and thus form a chitosan-collagen composite material; or (c) mixing an acidic solution of chitosan and an acidic solution of collagen and then evaporating the solvent from the mixed acidic solution to form a chitosan-collagen composite material; or (d) mixing an acidic solution of chitosan and collagen with a base or salt to precipitate the chitosan and collagen in the solution phase and thus form a chitosan-collagen composite material; in any case the resulting chitosan-collagen composite material optionally being further subjected to a crosslinking treatment.

8. A process for producing a substratum for cell culture, as claimed in Claim 7, wherein (a) the collagen is shaped, or wherein (b) the chitosan is shaped.

9. A process for producing a substratum for cell culture, as claimed in any one of Claims 7 to 9, wherein the chitosan is selected from chitosan, carboxymethylchitosan, glycol chitosan and succinylchitosan, and/or the collagen is selected from succinylcollagen, methylated collagens and enzyme-solubilized collagens.

10. The use of a substratum as defined in any one of Claims 1 to 6 in cell structure.

FIG. 1

# FIG. 2

EP 0 318 286 A2

# FIG. 3

EP 0 318 286 A2

# FIG. 4

# FIG. 5

EP 0 318 286 A2

FIG. 6

# FIG. 7

# FIG. 8

EP 0 318 286 A2